# EUROPEAN PATENT APPLICATION

(11) **EP 1 418 431 A1**
(43) Date of publication of application: **12.05.2004**
(21) Application number: 02079662.9
(22) Date of filing: 07.11.2002
(51) Int. Cl.: G01N 33/564, G01N 33/574, A61P 29/00, A61P 37/00, A61P 35/00

(54) **Nuclear pore protein Nup88 as a novel target**

(71) Applicant: UMC St. Radboud, 6500 HB Nijmegen (NL)
(72) Inventor: Takahashi, Nozomi, 6581 DP Malden (NL); van den Berg, Wim B., 6584 CB Molenbeek (NL)
(74) Representative: van Westenbrugge, Andries

(57) **Abstract**

The present invention relates to the use of nucleoporin 88 as a novel target within the signaling pathway of NF-κB. It provides a new therapeutic approach for a broad range of diseases in which NF-κB is involved. This is for instance the case for inflammatory diseases, such as rheumatoid arthritis, asthma, inflammatory bowel disease, systemic inflammatory response syndrome etc. and cancer. Pharmaceutical compositions which comprise a compound which interferes with nucleoporin 88 activity or expression are also part of the invention.

## Description

### Field of the invention

The present invention relates to the nuclear pore protein Nup88. More specifically it relates to the use of Nup88 as a disease marker and its use in diagnosis and therapy.

### State of the art

Nuclear pore complexes are large elaborate macromolecular structures that mediate the bidirectional nuclear-cytoplasmic transport. In vertebrates, nuclear pore complexes comprise 50 to 100 proteins that are called nucleoporins (Nup). Only about 23 of these nucleoporins have been identified so far. One of these is Nup88, a predominantly cytoplasmic protein that is found to be associated in a dynamic subcomplex with the oncogenic nucleoporin CAN/Nup 214. Nup88 is a putative tumor marker, which is over-expressed in tumors and embryonic tissue. The functional significance of this overexpression remains unknown, despite several studies.
In V. Gould *et al.,* Am J Pathol 157:1605, 2000 it is described how polyclonal antiserum to Nup88 immunoreacts in tissue sections of malignant tumors of many types, indicating that Nup88 is a molecule of exceedingly wide distribution which is over-expressed during tumorigenesis and development. About the role of Nup88 one can only speculate.
N.Martinez *et al.,* Cancer Res 59:5408, 1999 describe that Nup88 is overexpressed in a large proportion of ovarian tumor cells. It is postulated that the protein is a potential marker of malignancy. However, the reasons for overexpression of Nup88 are unknown.
Uv *et al.,* Genes & Development 14: 1945, 2000 describe a *Drosophila* gene *members only (mbo)* which encodes a *Drosophila* nucleoporin Dnup88 which shows homology to the mammalian Nup88. The authors postulate that Dnup88 is required for nuclear import of selected proteins.

*Bastos et al.,* J Cell Biol 137:989, 1997 describe the characterisation of a rat nucleoporin called Nup84, which is associated with CAN/Nup214. Nup84, which is supposed to be the rat equivalent of Nup88, is proposed to function in the assembly of CAN/Nup214 on the cytoplasmic face of the nuclear pore complex (NPC). Its role may be to organise docking sites for incoming nuclear proteins at the cytoplasmic periphery of the NPC.
To date no clear and conclusive evidence is available in regard to the precise function of vertebrate Nup88 and its possible role in diseases.

### Detailed description

The present invention relates in one aspect to the use of the nucleoporin Nup88 as a marker for inflammatory diseases, immune disease and metastatic tumors.

In this context, the term "Nup88" refers to a protein or polypeptide with an amino acid sequence which is substantially similar to the amino acid sequence as published in Fomerod,M. et al. EMBO J. 16 (4), 807 (1997) (GeneBank Acc. No. NP_002525). The term "Nup88" is thus also used for nucleoporin 88, nucleoporin 88kD(a), nuclear pore protein 88 as well as for their non-human mammalian homologues and includes allelic forms and muteins of these protein or polypeptide comprising one or more amino acid substitutions, deletions and/or insertions. The term "Nup88" as used in this context thus comprises a protein or polypeptide preferably having at least 63% amino acid identity with the amino acid sequence assigned to human Nup88 (GeneBank Acc. No. NP_002525), more preferably having at least 75, 80, 90, 95 or 99% amino acid identity with the amino acid sequence of human Nup88 (GeneBank Acc. No. NP_002525). Included in the term "Nup88" are also proteins of the same or similar sequence as a native Nup88 protein, but lacking amino acid sequences at either or both of its N-terminal and C-terminal ends. The truncation mutants preferably comprise at least 50-60 amino acid residues more preferably 90-100 amino acid residues, and most preferably at least 150 amino acid residues of the untruncated Nup88 protein, or variant thereof, while retaining at least one activity of the Nup88 protein. Such truncated Nup88 preferably have at least 63% amino acid identity with the amino acid sequence of human Nup88 (GeneBank Acc. No. NP_002525). preferably having at least 75, 80, 90, 95 or 99% amino acid identity with the amino acid sequence of human Nup88 (GeneBank Acc. No. NP_002525).
Any protein, polypeptide or truncated mutant comprised within the term "Nup88" preferably has at least one biological activity of the native Nup88 protein defined by the amino acid sequences of human Nup88 (GeneBank Acc. No. NP_002525). Preferably, the biological activity of the Nup88 protein for use in the present invention at least comprises one or more of: (1) the ability to to be used to determine the sub-cellular localization of the activated NF-kappa B (NF-κB) and (2) the ability to mediate NF-κB dependent gene activation as described in the Examples herein.
The terms "NF-kappa B" and "NF-κB" refer to the transcription factor NF-kappa B which has a central role in the regulation of intracellular signals in many cell types and which is e.g. described in US patent 5,804, 374. The term "activated NF-kB" refers to NF-kB which has its nuclear localization signals (NLS) exposed.
The amino acid identity between a polypeptide comprised in the term "Nup88" and the sequence human Nup88 (GeneBank Acc. No. NP_002525) may be readily calculated by known methods, including but not limited to those described in (Computational Molecular Biology, Lesk, A. M., ed., Oxford University Press, New York, 1988; Biocomputing: Infomatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988). Preferred methods to determine identity are designed to give the largest match between the sequences tested. Methods to determine identity are codified in publicly available computer programs. Preferred computer program methods to determine identity and similarity between two sequences include, but are not limited to, the GCG program package (Devereux, J., et al., Nucleic Acids Research 12 (1):387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity. Preferred parameters for polypeptide sequence comparison include the following: 1) Algorithm: Needleman and Wunsch, J. Mol. Biol. 48:443-453 (1970) Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for peptide comparisons (along with no penalty for end gaps).

In this context, "inflammatory disease" refers to a disease, which is characterised by local or systemic evidence of inflammation and resulting in tissue or organ damage. In this context, the term "immune disease" refers to a disease originating from inappropriate immune responses and includes autoimmune diseases and allergies. These two categories of diseases, inflammatory disease and immune disease, largely overlap. They are exemplified by and include but are not limited to diseases such as asthma, juvenile diabetes mellitus, myasthenia gravis, Graves' disease, rheumatoid arthritis, allograft rejection, inflammatory bowel disease, systemic inflammatory response syndrome, multiple sclerosis, psoriasis, lupus erythematosus, systemic lupus erythematosus, diabetes, multiple sclerosis, contact dermatitis, rhinitis.

This aspect of the invention is particularly useful for the diagnosis of rheumatoid arthritis. It may advantageously be used for diagnosing rheumatoid arthritis in its early stage, i.e. with disease duration of less than about 1-1.5 years from the onset of first symptoms.

In this context, the term "metastatic tumors" refers to tumor cells that exhibit characteristics of metastasis: capacities to migrate and invade verified by cytological or histological evidence.

In another aspect the present invention relates to a method for screening for or diagnosis of an inflammatory or immune disease or a metastatic tumor in an individual. The method comprises the detection of the presence of Nup88 in a tissue from an individual. The presence of Nup88 in the tissue is indicative of an inflammation, pathological immune activation, or presence of tumor. The tissue which is subjected to screening is preferably isolated from the individual.

In the tissues of healthy individuals Nup88 will not be present, or at very low levels, which are under the detection levels of conventional immunohistochemical techniques, such as immunofluorescence and immunodetection, see e.g. the Examples. In the case of disease, Nup88 will be overexpressed. In the case of inflammatory or immune diseases, the intensity and extensiveness of the Nup88 signal correlates with the disease activity.
In metastatic tumors, Nup88 levels will not necessarily be different from levels in other type of tumor cells, but its localisation will. In metastatic tumor cells, Nup88 will primarily be detected around the nuclear membrane extending to the cytoplasm, whereas in non-metastatic tumor cells it will primarily be detected in the cytoplasm.

The detecting step may be carried out in a sample of tissue still present in the individual, or it may be isolated from the individual, using methods known in the art for the detection of an intracellular protein. An intrabody (intracellularly made antibody) may be used, together with an appropriate delivery system. The construction of intrabodies has been described, e.g. in US patent 6,004,940 and in WO 01/48017. If a technique is used which exposes the intracellular, an antibody directed against Nup88 may conveniently be used. Methods for generating polyclonal and monoclonal antibodies are referred to in the Examples and are generally known in the art (see e.g. Harlow and Lane (1988) *Antibodies, A Laboratory Manual,* Cold Spring Harbor Laboratory, Cold Spring Harbor Laboratory Press, New York).The antibody may be used as such, but preferably the antibody is labeled with a detectable label. Suitable antibody labels are known to the person skilled in the art and include, but are not limited to, radioactive labels, electron dense labels, enzymatic labels and fluorescent labels. In a preferred embodiment, see Examples, a fluorescent marker is used.
The extent of reaction may be visually scored by grading, e.g. : (+):1% positive cells. 1+: 1-5% positive cells, 2+: 6-15% positive cells, 3+: 16-50% positive cells, 4+: 51-95% positive cells, 5+: 96-100%. Intensity of reaction may be als be scored visually by grading :weak, moderate, strong, all in comparison to a control of healthy individuals.

The inventors also found that deletion of the Nup88 molecule blocks the activity of transcription factor NF-kappa B. NF-kappa B acts as an intracellular messenger in many cell types where it mediates gene expression. It is involved in many immune and inflammatory diseases. Therefore, a further aspect of the invention is the use of a compound which interferes with Nup88 activity or Nup88 expression for the preparation of a medicament. In particular, the compound may be used for the treatment of NF-kappa B related diseases. More particular, it may be used for the treatment of an inflammatory or immune disease.

In yet another aspect of the invention, a pharmaceutical composition or formulation comprising a compound which interferes with Nup88 activity or expression is disclosed. The compound will generally be present in the pharmaceutical composition in association with a carrier and, optionally, a pharmaceutically acceptable adjuvant.

The pharmaceutical composition or formulation of the present invention may be administered in a number of ways depending upon whether local or systemic treatment is desired and upon the area to be treated. Administration may be topical pulmonary, e.g., by inhalation or insufflation of powders or aerosols, including by nebulizer; intratracheal, intranasal, epidermal and transdermal, oral or parenteral. Parenteral administration includes intravenous, intra-arterial, subcutaneous, intraperitoneal or intramuscular injection or infusion; or intracranial administration.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s).

A compound which interferes with Nup88 activity or expression must be present in a therapeutically effective amount to be able to interfere. The interfering compound will decrease Nup88 activity or expression by at least 10%. Preferably, Nup88 activity is decreased by at least about 20 to 40%, more preferably, it is decreased by at least 40 to 60% or 60 to 70%, most preferably it is decreased by at least about 70 to 80%, 80 to 90%. All percentages are in relation to the activity when the interfering compound is not present.

Antibodies, preferably intracellular antibodies (intrabodies), functional antibody fragments and chemical or peptide inhibitors are examples of compounds that may be used to interfere with Nup88 activity Methods to identify inhibitors, such as peptides, peptide-sequences, peptide-like molecules and non-peptide molecules that bind to Nup88 and that may be identified using methods known in the art per se, as e.g. described in US 6,180,084 which incorporated herein by reference. Such methods include e.g. screening libraries of peptidomimetics, peptides, DNA or cDNA expression libraries, combinatorial chemistry and, particularly useful, phage display libraries. These libraries may be screened for Nup88 binding molecules by contacting the libraries with substantially purified Nup88, fragments thereof or structural analogues thereof.

A compound which interferes with Nup 88 expression may interfere at the DNA level, by interfering with e.g. replication and/or transcription. Alternatively, it interferes at the RNA level, e.g. by interfering with the translocation of the RNA to the site of protein translation, or with the translation of protein from the RNA, or with the splicing of the RNA to yield one or more mRNA species The overall effect of such interference with Nup88 expression is a decrease (inhibition) in the expression of the Nup88 gene. Interference on RNA level, in particuar on mRNA level is preferred, as it allows interference at the high expression level without affecting the very low physiological expression level. The disease state is associated with high expression level of Nup88 which is reflected by high level of its mRNA. Methods and materials for determining the expression of RNA in tissue sample are known the art (see e.g. Sambrook and Russel (2001) "Molecular Cloning: A Laboratory Manual (3^{rd} edition), Cold Spring Harbor Laboratory, Cold Spring Harbor Laboratory Press, New York).
An antisense oligonucleotide or nucleic acid which is complementary to at least a functional part of a Nup88 RNA may be used. The complementary part of the antisense oligonucleotide or nucleic acid is preferably at least 15, 20, 25, 30, 35, 40, 45 or 50 bases long. Preferably, the complementary part of antisense oliogotide or nucleic acid has less than 30, 25, 10, 5% mismatches, more preferably the complementary part of the antisense nucleic has no mismatches with the corresponding sequence in the Nup88 mRNA. Alternatively, the molecule that reduces or inhibits the expression of the Nup88 protein is a protein or small molecule that interferes with expression of the Nup88 protein by binding to a functional part of the Nup88 mRNA, Nup88 transcription regulatory sequences, or other factors required for the regulation of transcription of the Nup88 gene or for Nup88 mRNA stabilization or translation. In a preferred embodiment, RNA interference (RNAi) is used to interfere at the RNA level, using small interfering (si)RNA targeting appropriate sequence on Nup88 mRNA. In another preferred hairpin RNA (shRNA) with appropriate expression vector is used in RNAi to interfere at the RNA level. There are several possible target sequences on Nup88. In one preferred embodiment, RNAi is used, using 5' -aatgctttgtggagcacattctt-3', which is nucleotide 235-257 of mouse Nup88, as a target sequence. Other suitable sequences for mouse Nup88 include but are not limited to 5'-aaggcagcgccttcttggtggtt-3'. Suitable target sequences for human Nup88 include but are not limited to 5'-aaccgaagctgagaaaccagctt-3', 5'-aagacagctccttcttagtcgtt-3' and 5' - aaaagtcctgggattccaggatt-3'.

### EXAMPLES

### MATERIALS AND METHODS

Patients. The human synovial tissue collection (n=40) comprised samples of 25 female and 15 male patients with a mean age of 50,5 years (range: 18-77 years (yrs.)) with either long-standing RA (n=10) (disease duration: range: 5-16 years (mean: 9.56 yrs.)) or early RA (eRA) (n=10) (disease duration: < 1,5 yrs., as measured from the first clinical signs of polyarthritis, mean: 12 months), OA (=10) and synovial samples from healthy controls (n=10). Patients with RA meet the American College of Rheumatology criteria (The American Association 1987 revised criteria for the classification of rheumatoid arthritis. Arthritis Rheum 1988;31:315-24. Arnett F, Edworthy S, Bloch D et al.)
Mean disease activity score (DAS) of long-standing RA patients was 3.2 (range 2.2-5.5). Patients with eRA were naive/non-treated and had a mean DAS of 4.68 (range 3.11-7.61). Synovial samples from eRA patients were obtained by miniathroscopy from the metacarpophalangeal (MCP) joint II of the clinically dominant hand under local anesthesia. Synovial specimens from long-standing RA and OA were obtained at synovectomy or open knee surgery and normal tissue samplings from healthy controls undergoing trauma surgery. The specimens were immediately covered with tissue embedding medium (OCT, GSV1, Slee Technik GmbH, Mainz, Germany), frozen in liquid nitrogen and maintained at - 80 °C.
The protocol of tissue sampling and analysis was approved by the ethic committee of the Heinrich-Heine University of Düsseldorf and written consent was obtained from every in- and out-patient of the Center for Rheumatology Düsseldorf.

**Antibodies.** Polyclonal antibody against human Nup88 was raised in rabbit as described in M. von Lindern et al. Mol Cell Biol 1992 12: 3346. Antibody directed against nuclear localization signal (NLS) of the p65 subunit ofNF-κB was purchased from Chemicon International (Temecula, CA) and Pharmingen (Franklin lakes, NJ).

**Immunohistochemistry.** The first section of each series was stained with a standard laboratory hematoxylin and eosin stain for histologic evaluation and morphology assessment. Negative control experiments were performed by using the primary antibodies with blocking reagents. Frozen synovial biopsies were cut with a cryostat into sections 7 µm thick. Immunohistochemistry was performed on cryosections by using anti-Nup88 polyclonal antibody in a dilution of 1:1000 and/or NF-κB diluted 1:75 in APAAP technique, based on a standard protocol (DAKO Diagnostika GmbH, Hamburg, Germany). As the second antibody we used a commercial goat-mouse-rabbit antibody (DAKO, K 4017). A fuchsin substrate solution (DAKO, K 0624) was used to develop the reactions, supplemented with 0.2. mM levamisole (DAKO, X 3021) to block endogenous alkaline phosphatase. For doublestaining we used the Universal DAKO EnVision Doublestain System (DAKO, K 1395), which permits the simultaneous demonstration of two antigens within one specimen by double immunoenzymatic staining. Additionally, for blood vessel characterization and its identification we performed Collagen IV (Col IV) staining (Immunotech, Marseille, France). The extent of reaction was visually scored by grading: (+):1% positive cells. 1+: 1-5% positive cells, 2+: 6-15% positive cells, 3+: 16-50% positive cells, 4+: 51-95% positive cells, 5+: 96-100%. Intensity of reaction was scored by grading visually :weak, moderate, strong, all in comparison to the control.

**Immunofluorescence.** The same sections were used as described in the previous paragraph. We have used Texas Red (TXR)-labeled goat anti-rabbit antibody and fluorescein (FITC)-labeled goat anti-mouse antibody for the immunofluorescent detection of Nup88 and NF-κB. Intensity of reaction was scored by grading visually: weak, moderate, and strong, all in comparison to the control.

**RNA interference.** Nup88 specific small interfering siRNA (siRNA) and NF-κBp65 specific siRNA were designed according to the predicted target sequence that can be recognized by RNA interfering silencing complex (RISC) using full length mouse Nup88 (also called preimplantation protein 2, GenBank Acc. No.: XM_109854), full length mouse (m) NF-κBp65 (GenBank Acc. No.: BC003818) and human (h) NF-κBp65 (GenBank Acc. No.: NM_021975). The following target sequences (nucleotide number from the ORF start codon) were chosen for each molecule. mNup88: 235-257, mNF-κBp65:1363-1385, hNF-κBp65: 1366-1388. Human sequence for NF-κBp65 differed from mouse only with two nucleotides. Both sense and anti-sense single stranded (ss) RNA corresponding to the target sequences were synthesized and HPLC purified by RNA-TEC (Leuven, Belgium) and annealed to generate double stranded (ds) RNA.

**Luciferase reporter gene assay.** NIH3T3 cells were transfected with a plasmid construct carrying five consensus NF-κB binding site followed by a luciferase reporter gene, and a stable clone with the highest response to TNF and IL1 was selected (NF-κB-luc/3T3). NF-κB-luc/3T3 cells were seeded at the concentration of 1.5x10⁵ cells/ ml in a crystal 96 well microtiter plate 18-24 hours before the transfection. The cells were transfected with dsRNA (10nM) using oligofectamine (Invitrogen, Carlsbad, CA) according to the manufacturer's instruction. The cells were stimulated with either TNF (10ng/ml) or IL1 (10ng/ml) at 24 hrs and 48 hrs after the transfection, and assayed for luciferase activity 6hrs after the stimulation.-Briefly, luciferase substrate was added to the culture medium and the resulting light emission was measured by Fluorostar fluoro / luminometer.

### Example 1

### Expression of Nup88 in synovial tissue samples from rheumatoid arthritis (RA) patients.

The immunolocalization of Nup88 was performed on a collection of synovial tissue samples (n=10) from patients with long-standing RA (RA ) as described in Materials & Methods. All samples from the RA patients showed strong and distinct expression of Nup88 at the synovial lining which correlated with the degree of synovial hyperplasia and inflammation.

### Example 2

### Expression of Nup88 in synovial tissue samples from early RA patients

The immunolocalization of Nup88 was performed on a collection of synovial tissue samples (n=10) from patients with early RA (eRA) as described in Materials & Methods.
All samples from the patients with early RA also showed strong and distinct expression of Nup88 at the synovial lining, which correlated with the degree of synovial hyperplasia and inflammation. In synovial samples of eRA patients with severe disease activity expression of Nup88 was increased and highly correlated to the degree of cell infiltration. The signal was more intense than in patients with long-standing RA.

### Comparative example 3

### Expression of Nup88 in synovial tissue samples from patients with ostearthritis.

The immunolocalization of Nup88 was performed on a collection of synovial tissue samples (n=10) from patients with osteoarthritis (OA) as described in Materials & Methods. The samples from the patients with osteoarthritis showed much weaker signal in their surface lining than the signal observed in patients with RA or eRA. This correlated with a mild degree of inflammation, often associated with OA.

### Comparative example 4

### Expression of Nup88 in synovial tissue samples from healthy controls.

The immunolocalization of Nup88 was performed on a collection of synovial tissue samples (n=10) from healthy controls as described in Materials & Methods. The samples from the healthy controls showed no expression of Nup88 at all.

Examples 1 to 4 show that the expression of Nup88 is indicative of disease and that the intensity and extensiveness of the Nup88 signal correlates with the disease activity.

### Example 5

Double immunostaining and double-immunofluorescence was performed on a collection of synovial tissue samples from RA patients (n=10) using antibodies against Nup88 and NF-κB p65 NLS (revealed in activated NF-κB) as described in Materials and Methods. Nup88 and activated NF-κB co-localized in distinct areas: in the synovial lining and infiltrating cells. Co-localization was found both in cytoplasm and nucleus. As the activated NF-κB is usually not found in cytoplasm, this example shows that Nup88 determines the sub-cellular localization of the activated NF-κB.

### Example 6

### Deletion of Nup88 molecule reduces activity of NF-kB

Depletion of Nup88 by RNA interference by using small interfering RNA (siRNA) directed against Nup88 significantly reduced the NF-κB dependent gene activation measured by the luciferase reporter gene assay. This inhibitory effect was comparable to that of the depletion of NF-κB itself by using siRNA directed against NF-κB.

## Claims

1. The use of a compound which interferes with Nup88 activity or expression for the preparation of a medicament.

2. The use according to claim 1 for the preparation of a medicament for the treatment of a NF-kappa B related disease.

3. The use according to claim 1 or 2 for the treatment of an inflammatory disease, an immune disease or metastatic tumors.

4. A method for preventing or treating a disease which method comprises interfering with the activity and/or expression of Nup88.

5. A method according to claim 4 wherein the disease is an inflammatory disease, an immune disease, or a metastatic tumor.

6. Pharmaceutical composition comprising a compound which interferes with Nup88 activity or expression in association with a pharmaceutically acceptable carrier and, optionally, a pharmaceutically acceptable adjuvant.

7. The use of Nup88 as a marker for an inflammatory disease, an immune diseases or metastatic tumors.

8. The use according to claim 7 wherein the disease is rheumatoid arthritis.

9. The use according to claim 8 wherein the rheumatoid arthritis is in its early stage.

10. A method for screening for or diagnosis of an inflammatory disease, or an immune disease in an individual, which method comprises detecting the presence of Nup88 in a tissue from the individual, whereby its presence is indicative of the disease.

11. A method for screening for or diagnosis of the presence of a metastatic tumor in an individual, which method comprises evaluating the localization of Nup88 in a tumor cell from the individual, whereby Nup88's localization around the nuclear membrane is indicative of the metastatic character of the tumor cell.

12. A method according to claim 10 or 11, wherein the detecting step is carried out in a sample of tissue isolated from the individual.

13. A method according to claims 10-12, wherein the detection step comprises exposing the tissue to a compound which interacts with Nup88, wherein the compound is optionally labeled with a detectable label.
